# EUROPEAN PATENT APPLICATION

(11) **EP 2 433 615 A1**
(43) Date of publication of application: **28.03.2012**
(21) Application number: 10777250.1
(22) Date of filing: 12.05.2010
(51) Int. Cl.: A61K 8/37, A61K 8/97, A61K 8/25, A61Q 5/00

(54) **METHOD FOR PRODUCING A HAIR SHINE COSMETIC, AND HAIR SHINE COSMETIC PRODUCT**

(30) Priority: 20.05.2009 BR PI0901649
(71) Applicant: Gonçalves Da Silva, Denivaldo, 09715-240 São Bernardo do Campo SP (BR)
(72) Inventor: Gonçalves Da Silva, Denivaldo, 09715-240 São Bernardo do Campo SP (BR)
(74) Representative: Zenz
(86) International application number: PCT/BR2010/000151
(87) International publication number: WO 2010/132965

(57) **Abstract**

A hair shine cosmetic product and a method for producing same are disclosed. The product is composed of sunflower glycerides, water, sunflower seed extract, a plant mixture, preservatives, citric acid and a fragrance, besides silica or Antil 200 (hydrogenated and polyethoxylated glyceryl palmate with 200 ethylene glycol units, combined with polyethoxylated glyceryl cocoate with 7 ethylene glycol units). The production method comprises weighing the components, mixing the sunflower glycerides, water, sunflower seed extract and plant mixture for at least 30 minutes under constant stirring, slowly spraying the silica or Antil 200, heating the resultant mixture to 50°C and keeping it at this temperature for at least 60 minutes, reducing the temperature to 25-30°C, adding the preservative, citric acid and fragrance.

## Description

### TECHNICAL FIELD

The present invention, the manufacturing process of a cosmetic product and its resulting product, was primarily conceived to promote hair shine. The product is usable particularly after the bath, after the hair has been washed with an appropriate shampoo and conditioner, and may be applied to wet or dry hair. The process for the obtention of a cosmetic product and its resulting product presents quite reasonable costs and its proposed structure is substantially simplified and efficient.

### TECHNIQUE FUNDAMENTALS

As is widely known by manufacturers and other professionals in the cosmetics area, and especially by the female public, the consumer market is abundantly supplied with hair cleaning and treatment products such as shampoos, conditioners, revitalizers, moisturizers and other hair care products.

As a matter of fact, a shampoo is a product that has the intrinsic aim of adequately caring for hair, i.e., it is used mainly to remove oiliness from hair and dirt and dead skin
remains from the scalp , which, over time, bind to hair and can provoke bad odor and itching, as well as scalp diseases and hair loss.

Hair conditioner is usually applied on hair after shampooing, in order to improve hair texture and general aspects.

Most cosmetic products such as shampoos, conditioners and congeners actually present in the consumer market contain silicone in their formulations, since silicone provides shine and protection and eliminates water from hair.

However, silicone is an element that may be retained in the scalp and/or accumulate on hair. This retention can impede good hair breathing, weakening the roots, causing hair to become visibly damaged and dry.

The lack of silicone in the formulation results in the lack of features capable of offering appropriate hair shine and protection when the products mentioned are used.

Moreover, to obtain the appropriate hair shine and protection, the consumer market offers products such as hair creams, moisturizers and structure revitalizers, which are commercialized separately.

In this case, the user must purchase a product she may not need to obtain the shine desired for her hair.

### DETAILED DESCRIPTION OF INVENTION

The present Invention Patent has as its main objective the process of obtaining a product capable of promoting greater hair shine and beauty, particularly when applied after hair washing, and which can be applied to wet or dry hair, promoting a stage of substantial improvement to the hair conditions.

Therefore, this "MANUFACTURING PROCESS FOR A COSMETIC PRODUCT TO PROMOTE HAIR SHINE AND COSMETIC PRODUCT" process must be highlighted among its congeners and be personalized in the consumer market because it constitutes a process that presents a plurality of elements which, when combined in the correct proportions, make a product that presents innovative features compared to its congeners.

The ingredients and percentages needed for the production of the product claimed in the manufacturing process of a cosmetic product are the following:

| | | |
|---|---|---|
| Rotação Deva Brilho Soro de Leite WS | | |

| Item | Ingredient | % |
|---|---|---|
| 1 | Sunflower Glycerics * | QS |
| 2 | Deionized Water | ∼2.00 |
| 3 | WS Sunflower Seed Extract | 0.20 |
| 4 | WS Deva Botanical Mixture | 0.20 |
| 5 | Silica | 1.00-3.00 |
| 6 | Preservative | 1.10 |
| 7 | 25% Citric Acid Solution | 0.10 |
| 8 | Fragrance | 0.60 |
| | Total | 100.00 |

The mixing process must follow the stages described below; i.e., first all the items needed for production are pre-weighed, next the sunflower glycerics are introduced into a lined mixing tank equipped with a turbine stirrer. Mild agitation is used and deionized water is slowly added, along with WS sunflower seed and WS Botanical mixture. When all these ingredients are in the tank, they are mixed for at least 30 minutes.

The next procedure follows the slow spraying of silica in the amount described, and the mixture is then heated to 50°C.

After reaching 50°C, the temperature is maintained for at least 60 minutes; a homogenizer is used to form a consistent solution.

The next step is to reduce the solution temperature to between 25°C - 30°C, stirring it concomitantly and moderately.

While temperature is between 25°C - 30°C, the preservative, the 25% citric acid solution and the fragrance are slowly added, one at a time.

Mix for 30 more minutes before separating samples for mixture analysis.

In this manner the manufacturing process yields a product that offers the first "silicone-free" Brilho Soro de Leite; the first water-soluble Brilho Soro de Leite - not formed on hair; sunflower glycerics; and the Hydrophyl element (HLB 8-10) derived from oil glycerics with high oleic sunflower.

The other process formulation is described below and presents substantially the same procedures, with different materials and their required procedures, i.e. it presents a plurality of elements, which are the following when combined in the correct proportions for the cosmetic product production to promote hair shine:

| | |
|---|---|
| Rotação Deva Brilho Soro de Leite WS | |

| Formula A | |
|---|---|
| Ingredient | % |
| Sunflower Glycerics * | 93.30 |
| Deionized Water | ∼2,00 |
| WS Sunflower Seed Extract | 0.20 |
| WS Deva Botanical Mixture | 0.20 |
| Hydrogenated Glyceryl Palmate PEG-200 | 2.50 |
| (e) Glyceryl Cocoate | |
| Commercial Name: Antil 200 - Evonik Industries | |
| Preservative | 1.10 |
| 25% Citric Acid Solution | 0.10 |
| Fragrance | 0.60 |
| Total | 100.00 |

The new characteristics of the product obtained through this manufacturing process are described below:
   First "silicone-free" Brilho Soro de Leite; the first water-soluble Brilho Soro de Leite - not formed on hair; sunflower glycerics; and the Hydrophyl element (HLB 8-10) derived from oil glycerics with high oleic sunflower.

It is important to understand that although not detailed, this invention's applications are not limited to the details and stages described herein. The invention is capable of other modalities and can be practiced or executed in several manners. It should be understood that the terminology used herein is for description and not for limitation purposes.

## Claims

1. "MANUFACTURING PROCESS FOR A COSMETIC PRODUCT TO PROMOTE HAIR SHINE," **characterized by** the mixing process, which obeys the stages described below, i.e., first an accurate pre-weighing of all the items needed for the production of this product; next, sunflower glycerics are introduced in a lined mixing tank equipped with turbine stirrer; moderate stirring is employed and deionized water, WS sunflower seed extract, and WS Botanical mixture are slowly added to the mixture. With all these ingredients in the tank, the mixture is stirred for at least 30 minutes. The next procedure arises from the slow spraying of silica in the amount described and the mixture is heated to 50°C. After reaching 50°C, the temperature is kept uniformly at this temperature for at least 60 minutes; a homogenizer is used to form a consistent solution. The following step is reduction of the solution temperature, keeping it between 25°C - 30°C while stirring it concomitantly and moderately. Maintaining the temperature between 25°C - 30°C, the preservative, the 25% citric acid solution and the fragrance are slowly added, one at a time, mixing for 30 more minutes before separating
samples for mixture analysis.

2. "COSMETIC PRODUCT," **characterized by** the following necessary ingredients and percentages for the product's manufacturing process:
| | | |
|---|---|---|
| Rotação Deva Brilho Soro de Leite WS | | |
| Item | Ingredient | % |
|---|---|---|
| 1 | Sunflower Glycerics * | QS |
| 2 | Deionized Water | ∼2.00 |
| 3 | WS Sunflower Seed Extract | 0.20 |
| 4 | WS Deva Botanical Mixture | 0.20 |
| 5 | Silica | 1.00-3.00 |
| 6 | Preservative | 1.10 |
| 7 | 25% Citric Acid Solution | 0.10 |
| 8 | Fragrance | 0.60 |
| | Total | 100.00 |

3. "COSMETIC PRODUCT," in accordance with claim 2 above, and in an alternative formulation, **characterized by** the following necessary ingredients and percentages:
| | |
|---|---|
| Rotação Deva Brilho Soro de Leite WS | |
| Formula A | |
|---|---|
| Ingredient | % |
| Sunflower Glycerics * | 93.30 |
| Deionized Water | ∼2.00 |
| WS Sunflower Seed Extract | 0.20 |
| WS Deva Botanical Mixture | 0.20 |
| Hydrogenated Glyceryl Palmate PEG-200 | 2.50 |
| (e) Glyceryl Cocoate | |
| Commercial Name: Antil 200 - Evonik Industries | |
| Preservative | 1.10 |
| 25% Citric Acid Solution | 0.10 |
| Fragrance | 0.60 |
| Total | 100.00 |
